# EUROPEAN PATENT APPLICATION

(11) **EP 3 792 349 A1**
(43) Date of publication of application: **17.03.2021**
(21) Application number: 19800484.8
(22) Date of filing: 25.04.2019
(51) Int. Cl.: C12N 9/00, C07K 14/36, C12N 15/52, C12N 15/63

(54) **PEPTIDE MACROCYCLASE**

(30) Priority: 07.05.2018 JP 2018089287; 19.03.2019 JP 2019050797
(71) Applicant: National University Corporation Hokkaido University, Sapporo-shi, Hokkaido 060-0808 (JP)
(72) Inventor: WAKIMOTO, Toshiyuki, Sapporo-shi, Hokkaido 060-0808 (JP); KURANAGA, Takefumi, Sapporo-shi, Hokkaido 060-0808 (JP); MATSUDA, Kenichi, Sapporo-shi, Hokkaido 060-0808 (JP)
(74) Representative: Müller, Christian Stefan Gerd
(86) International application number: PCT/JP2019/017707
(87) International publication number: WO 2019/216248

(57) **Abstract**

A peptide cyclase that has the amino acid sequence represented by SEQ ID NO: 1 or a mutated sequence thereof, or a peptide cyclase that has an amino acid sequence encoded by a base sequence encoding the amino acid sequence represented by SEQ ID NO: 1 or a mutated sequence thereof; DNA encoding the peptide cyclase; a method for producing the peptide cyclase; and a method for producing a cyclic peptide using the peptide cyclase.

SEQUENCE LISTING

<110>  National University Corporation Hokkaido University

<120>  An enzyme cyclizing large peptides

<130>  674911

<150>  JP 2018-089287
<151>  2018-05-07

<150>  JP 2019-050797
<151>  2019-03-19

<160>  12

<170>  PatentIn version 3.5

<210>  1
<211>  451
<212>  PRT
<213>  Streptomyces albidoflavus

<400>  1

Val Gly Ala Glu Gly Ala Glu Arg Asp Ala Val Gly Ala Leu Phe Glu
1               5                   10                  15

Glu Leu Val Arg Glu His Arg Val Thr Gly Ala Gln Leu Ser Val Tyr
            20                  25                  30

Arg Asp Gly Ala Leu Ser Glu Tyr Ala Thr Gly Leu Ala Ser Val Arg
        35                  40                  45

Thr Gly Glu Pro Val Thr Pro Arg Thr Gly Phe Pro Phe Gly Ser Val
    50                  55                  60

Thr Lys Phe Leu Thr Ala Glu Leu Val Met Gln Phe Val Cys Asp Gly
65                  70                  75                  80

Asp Leu Asp Leu Asp Asp Pro Leu Ala Gly Leu Leu Pro Asp Leu Gly
                85                  90                  95

Arg Ala Ala Gly Pro Pro Leu Gly Thr Ala Thr Val Arg Gln Leu Leu
            100                 105                 110

Ser His Thr Ala Gly Val Val Asp Ser Ile Glu Tyr Asp Glu Met Arg
        115                 120                 125

Gly Pro Ser Tyr Arg Arg Phe Ala Ala Ala Cys Ala Arg Gln Pro Ala
    130                 135                 140

Leu Phe Pro Pro Gly Leu Ala Phe Ser Tyr Ser Asn Thr Gly Tyr Cys
145                 150                 155                 160

Leu Leu Gly Ala Val Ile Glu Ala Ala Ser Gly Met Asp Trp Trp Thr
                165                 170                 175

Ala Met Asp Ser Cys Leu Leu Arg Pro Leu Gly Ile Glu Pro Ala Phe
            180                 185                 190

Leu His Asp Pro Arg Pro Gly Gln Gly Gly Ala Ala Arg Pro Val Ala
        195                 200                 205

Glu Gly His Ala Leu Arg Ala Gly Gly Glu Arg Ala Glu His Val Asp
    210                 215                 220

His Met Ala Ser Leu Ser Leu Ala Ala Ala Gly Gly Leu Val Gly Ser
225                 230                 235                 240

Ala Thr Asp Leu Val Thr Ala Ala Arg Pro His Leu Ala Asp Arg Lys
                245                 250                 255

Thr Phe Ala Gln His Asp Leu Leu Pro Glu Asp Ala Val Leu Ala Met
            260                 265                 270

Arg Thr Cys Val Pro Asp Ala Glu Pro Phe Gly Leu Ala Asp Gly Trp
        275                 280                 285

Gly Leu Gly Leu Met Arg His Gly Thr Gly Asp Gly Ala Trp Tyr Gly
    290                 295                 300

His Asp Gly Ala Val Gly Gly Ala Ser Cys Asn Leu Arg Ile His Pro
305                 310                 315                 320

Asp Arg Ser Leu Ala Leu Ala Leu Thr Ala Asn Ser Thr Ala Gly Pro
                325                 330                 335

Lys Leu Trp Glu Ala Leu Val Ala Arg Leu Pro Glu Ala Gly Leu Asp
            340                 345                 350

Val Gly His Tyr Ala Leu Pro Val Pro Asp Ser Ala Pro Leu Ala Pro
        355                 360                 365

Asp Ala Gly His Leu Gly Thr Tyr Ala Asn Gly Asp Leu Glu Leu Met
    370                 375                 380

Val Thr His Asp Ala Ala Gly Asp Leu Phe Leu Thr Arg Glu Ser Tyr
385                 390                 395                 400

Ser Asp Tyr Arg Leu Ser Leu His Glu Asp Asp Leu Phe Val Ala Arg

405                 410                 415

Ser Gly Glu Pro Gly Ala Leu Pro Ile Thr Gly Arg Phe Val Arg Glu
            420                 425                 430

His Pro Ala Gly Pro Val Ala Leu Leu Gln Tyr Gly Gly Arg Ala Met
        435                 440                 445

His Arg Leu
    450

<210>  2
<211>  1356
<212>  DNA
<213>  Streptomyces albidoflavus

<400>  2
gtgggtgccg agggggcgga acgggacgcg gtcggcgcgc tcttcgagga gctggtgcgc      60

gagcaccggg tcaccggggc gcagctgtcc gtctaccgcg acggcgccct cagcgagtac     120

gccaccggcc tcgcctcggt ccgcaccggt gaaccggtca ccccccggac gggtttcccc     180

ttcggctcgg tgaccaagtt cctcaccgcc gagctggtca tgcagttcgt ctgcgacggc     240

gacctggacc tcgacgatcc cctcgccggg ctcctccccg acctggggcg cgccgccggc     300

ccgcccctcg gcaccgccac cgtccgccag ctcctcagcc acaccgccgg ggtggtggac     360

agcatcgagt acgacgagat gcgcggtccc tcctaccggc ggttcgccgc ggcgtgcgcc     420

cggcagcccg cgctcttccc gccgggcctc gccttctcct actccaacac cgggtactgc     480

ctgctgggcg cggtgatcga ggcggcgtcg gggatggact ggtggacggc gatggacagt     540

tgcctgctgc gtccgctcgg catcgagccg gccttcctgc acgacccgcg ccccggccag     600

ggcggcgccg cccggccggt ggccgagggc cacgcgctgc gcgccggcgg cgagcgggcc     660

gagcacgtcg accacatggc ctcgctctcg ctggccgccg ccggcgggct ggtcggcagc     720

gccaccgacc tggtcaccgc ggcccgcccg cacctggccg accggaagac cttcgcccag     780

cacgacctgc tccccgagga cgccgtcctc gccatgcgca cctgcgtgcc ggacgccgag     840

ccgttcggcc tggccgacgg ctgggggctg ggcctgatgc gccacggcac cggcgacggc     900

gcctggtacg gccacgacgg cgcggtgggc ggcgcctcct gcaacctccg tatccacccg     960

gaccgttcgc tggcgctggc gctgaccgcc aactccaccg ccgggccgaa gctgtgggag    1020

gcgctggtcg cgcggctgcc ggaggccggg ctcgacgtgg gccactacgc gctgcccgtc    1080

cccgactccg cgccgctggc cccggacgcc ggccacctcg gcacctacgc caacggcgac    1140

ctggagctga tggtgaccca cgacgccgcc ggcgacctct tcctgacccg cgagagctac    1200

tccgactacc gcctctccct gcacgaggac gacctcttcg tggcccggag cggcgagccc    1260

ggcgcgctcc cgatcaccgg ccgcttcgtc cgggagcacc cggccgggcc ggtcgccctg 1320

ctccagtacg gcggccgggc catgcaccgg ctctga 1356

<210> 3
<211> 30
<212> DNA
<213> Artificial Sequence

<220>
<223> Forward primer

<400> 3

ccggaattcc atatgggtgc cgagggggcg 30

<210> 4
<211> 27
<212> DNA
<213> Artificial Sequence

<220>
<223> Reverse primer

<400> 4

cccaagcttt cagagccggt gcatggc 27

<210> 5
<211> 432
<212> PRT
<213> Goodfellowiella coeruleoviolacea

<400> 5

Val Pro Asn Glu Gln Asp Leu Gly Ser Leu Leu Ala Arg Leu Ala Glu
1               5                   10                  15

Arg His Arg Val Pro Gly Ala Gln Leu Ser Val Cys His Asp Ser Ala
            20                  25                  30

Ser Val Thr Val Val Thr Gly Arg Glu Arg His Gly Ala Ala Thr Pro
        35                  40                  45

Val Thr Ala Arg Ser Ala Phe Pro Leu Gly Ser Leu Thr Lys Pro Phe
    50                  55                  60

Thr Ala Ala Leu Ala Met Val Leu Val Ala Asp Gly Asp Leu Asp Leu
65                  70                  75                  80

Asp Glu Pro Val Ala Gly Tyr His Pro Gly Val Thr Leu Arg Arg Leu
                85                  90                  95

Leu Ser His Thr Ala Gly Leu Glu Ser Asn Val Asp Glu Gln Ala Ala

            100                 105                 110

Ala Gly Val Pro Arg Arg Arg Trp Val Thr Arg Tyr Pro Ala Arg Leu
        115                 120                 125

Ala Pro Glu His Pro Pro Gly Thr Val Phe Ser Tyr Ser Asn Val Gly
    130                 135                 140

Tyr Leu Leu Ala Gly His Leu Val Glu Glu Val Thr Gly Leu Asp Trp
145                 150                 155                 160

Ala Glu Ala Val Glu Ser Val Leu Leu Arg Pro Leu Gly Ile Thr Pro
                165                 170                 175

Ala Phe Val Val Gly Ala Ala Gly Ser Arg Gly Val Val Cys Gly His
            180                 185                 190

Val Leu Thr Asp Asp Arg Val Leu Pro Val Ala Glu Gln Val Met Pro
        195                 200                 205

Glu Val Glu Ala Pro Asn Gly Ala Leu Ala Leu Ser Ser Ala Asp Leu
    210                 215                 220

Leu Ala Phe Ala Ala Leu Phe Ser Ala Asn Ser Pro Ala Pro Asp Val
225                 230                 235                 240

Leu Asp Pro Ala Thr Ala Ala Leu Met Cys Gln Asp Gln Leu Ala Asp
                245                 250                 255

Val Arg Val Gly Pro Tyr Gly Met Ala Asp Gly Trp Gly Leu Gly Trp
            260                 265                 270

Ala Arg Tyr Arg Gly Ala Gly Ala Asp Trp Phe Gly His Asp Gly Thr
        275                 280                 285

Gly Asp Gly Thr Ser Cys His Leu Arg Phe Asp Pro Glu Arg Gly Thr
    290                 295                 300

Ala Val Ala Leu Thr Thr Asn Gly Ser Thr Gly Ala Ala Leu Trp Arg
305                 310                 315                 320

Glu Leu Cys Ala Glu Leu Ala Glu Arg His Val Leu Pro Pro Glu Trp
                325                 330                 335

Gly Arg Glu Pro Gly Glu Ala Thr Pro Val Ala Gly Pro Pro Asp Gly
            340                 345                 350

Ala Gly His Tyr Ala Asn Gly Pro Ala Glu Phe Val Val Thr Arg Gln
        355                 360                 365

Asp Asp Asp Gly Leu Ala Leu Val Leu Asp Gly Ala Gln Ala Val Pro
    370                 375                 380

Leu Thr Cys Leu Pro Asp Leu Arg Phe Gln Leu Arg Leu Gly Thr Thr
385                 390                 395                 400

Val Leu Ser Gly Arg Phe Leu Arg Asp Pro Asp Thr Gly Gly Ile Asp
                405                 410                 415

His Ile Gln Val Thr Gly Arg Leu Ala Arg Arg Arg Gln Val Thr Gly
            420                 425                 430

<210>  6
<211>  1299
<212>  DNA
<213>  Goodfellowiella coeruleoviolacea

<400>  6
gtgcccaacg agcaggatct cgggtcgctg ctggcccggc tggccgaacg ccaccgggtc      60

cccggcgccc agctcagcgt ctgccacgac agcgcctcgg tcacggtggt gaccggccgg     120

gaacgccacg gcgcggccac gccggtcacc gcgcggtcgg cgttcccgct ggggtcgctc     180

accaagccgt tcaccgcggc gctggccatg gtcctggtcg ccgacggcga tctggacctc     240

gacgagccgg tggccggcta tcaccccggg gtgaccctgc gccgcctgct cagtcacacc     300

gccgggctgg agtccaacgt ggacgaacag gccgcggccg gggtgccgcg ccgccgctgg     360

gtgacccgct acccggcgcg gctggccccg gagcacccgc cgggcacggt gttctcctac     420

tccaacgtcg gttacctgct ggccggccac ctggtggagg aggtgaccgg actggactgg     480

gccgaggcgg tggagtcggt gctgctgcga ccgctgggca tcaccccggc gttcgtggtg     540

ggcgccgcgg gcagccgtgg agtggtgtgc gggcacgtgc tgaccgacga ccgggtgctg     600

cccgtggccg agcaggtgat gcccgaggtc gaggcgccca acggcgcgct cgcgctgtcc     660

tcggccgacc tgctcgcctt cgcggcgctg ttctcggcga actccccggc ccccgacgtg      720

ctcgacccgg ccacggccgc gctgatgtgc caggaccagc tcgccgacgt gcgggtcggc     780

ccgtacggca tggccgacgg gtggggcctg ggctgggcgc gctaccgcgg cgcgggcgcc     840

gactggttcg gccacgacgg caccggcgac ggcacctcct gccacctgcg gttcgacccg     900

gagcgcggca cggccgtcgc gctcaccacc aacggctcga ccggcgcggc gctgtggcgg     960

gagctgtgcg ccgaactggc cgagcggcac gtgctgcccc cggagtgggg gcgcgagccc    1020

ggcgaggcca ccccggtcgc cgggccaccg gacggcgccg gccactacgc caacggcccc    1080

gccgagttcg tggtgaccag gcaggacgac gacggtctcg cgctggtgct cgacggtgcc    1140

caggccgtcc cgctgacctg cctgcccgac ctgcgcttcc agctccgcct cggcacgacg 1200

gtcctgagtg gacggttcct gcgcgacccg gacaccggcg gcatcgacca catccaggtg 1260

accggccgac tggcccggcg gcggcaggtg accggatga 1299

<210> 7
<211> 33
<212> DNA
<213> Artificial Sequence

<220>
<223> Forward primer

<400> 7
ccggaattcg tgcccaacga gcaggatctc ggg 33

<210> 8
<211> 31
<212> DNA
<213> Artificial Sequence

<220>
<223> Reverse primer

<400> 8
cccaagcttt catccggtca cctgccgccg c 31

<210> 9
<211> 439
<212> PRT
<213> Streptomyces noursei

<400> 9

Val His Gly Asp Ser Ala Asp Pro Ala Gly Tyr Gly Ala Gly Asp Gly
1               5                   10                  15

Ser Pro Leu Asp Leu Asp Leu Asp Arg Leu Ala Arg Asp Cys Asp Val
            20                  25                  30

Ala Gly Gly Gln Leu Ala Val His His His Gly Val Leu Glu Thr Trp
        35                  40                  45

Glu Phe Gly Glu Glu Glu Pro Gly Ala Gly Arg Pro Val His Ser Ala
    50                  55                  60

Thr Ala Phe Pro Tyr Gly Ser Thr Thr Lys Val Cys Thr Ala Thr Ser
65                  70                  75                  80

Val Leu Gln Leu Val Gly Asp Gly Asp Leu Asp Leu Asp Arg Pro Val
                85                  90                  95

Arg Glu Trp Leu Pro Glu Ala Gly Thr Val Pro Glu Ala Arg Arg Asp
            100                 105                 110

His Pro Ala Leu Ala Ala Thr Leu Arg Gln Leu Leu Ser His Thr Ala
        115                 120                 125

Gly Leu Pro Ser Asp His Asp Asp Ala Asp Ala Ser Ser Leu Arg Arg
    130                 135                 140

Trp Leu Thr Gly Phe Leu Ala Ala Pro Ala Asp Ala Arg Pro Ala Pro
145                 150                 155                 160

Gly Thr Phe Ser Tyr Ser Asn Val Gly Tyr Gly Ile Ala Gly Arg Val
                165                 170                 175

Val Glu Ser Val Thr Gly Leu Pro Trp Tyr Glu Ala Val Arg Asp Tyr
            180                 185                 190

Leu Leu Arg Pro Leu Gly Thr Ala Ile Thr Ala Leu Pro Ala Glu Pro
        195                 200                 205

Gly Ser Leu Pro Ala Gly Gly Leu Ala Gly Ser Ala Ala Asp Leu Val
    210                 215                 220

Arg Leu Gly Leu Leu His Ile Ala Glu Pro Gly Arg Pro Asp Leu Ala
225                 230                 235                 240

Asp Pro Ala Ala Leu Gly Glu Ala Thr Arg Pro Thr Ala Gly Ala Asp
                245                 250                 255

Pro Phe Gly Leu Ala Asp Gly Trp Gly Pro Gly Leu Gly His Phe Gly
            260                 265                 270

Pro Ala Glu Asp Arg Trp Leu Gly His Asp Gly Thr Leu Asp Gly Ala
        275                 280                 285

Thr Cys His Leu Arg Ile His Pro Arg Arg Gly Thr Val Ile Ala Leu
    290                 295                 300

Thr Thr Asn Ser Pro Thr Gly Gln Ala Leu Trp Asp Ala Val Val Gly
305                 310                 315                 320

Ala Leu Arg Asp Ala Gly Ile Asp Val Gly Val Tyr Arg Pro Ala Pro
                325                 330                 335

Pro Pro Ser Leu Ala Gln Gly Gly Ala Pro Ala Gly Ala Ala Ala Phe
            340                 345                 350

Ala Asp Cys Thr Gly Thr Tyr Arg Asn Gly Asp Leu Ala Val Thr Val
        355                 360                 365

Gly Leu Asp Gly Pro His Leu Val Leu Glu Leu Pro Gly Gly Ala Arg
    370                 375                 380

Glu Leu Ala Glu Pro Leu Ala Arg Arg Thr Phe Ala Ala Arg Gly Ala
385                 390                 395                 400

Gly Ala Phe Leu Gly Arg Phe Ala Val Asp Ala Arg Thr Gly Ala Val
                405                 410                 415

His Ala Leu Gln Tyr Ser Gly Arg Thr Leu Leu Arg Asp Thr Gly Arg
            420                 425                 430

Ala His Gly Ala Arg Pro His
        435

<210>  10
<211>  1320
<212>  DNA
<213>  Streptomyces noursei

<400>  10
gtgcacgggg actcagcgga tccagcgggg tacggcgccg gtgacggctc ccccctcgac      60

ctcgacctgg accgtctcgc ccgtgactgc gacgtggccg gcggacagct ggccgtccac      120

caccacggtg tgctggagac ctgggagttc ggcgaggagg aacccggcgc cggacggccg      180

gtgcactccg cgacggcctt cccctacggg tcgacgacca aggtgtgcac cgccacctcc      240

gtcctgcaac tggtcggcga cggcgacctg gacctcgacc ggcccgtacg ggaatggctc      300

cccgaggccg ggacggttcc cgaagcgcgg cgcgaccacc cggccctcgc cgccaccctc      360

cgccaactcc tcagccacac cgccggactg ccctccgacc acgacgacgc ggacgcctcc      420

tcgctgcgcc gctggctgac cggcttcctg gccgcaccgg ccgacgcccg gcccgccccg      480

ggcaccttct cctactccaa cgtcggctac ggcatcgccg gacgcgtcgt ggagtccgtc      540

accggactgc cctggtacga ggcggtacgg gactacctcc tgcgcccgct gggcaccgcc      600

atcaccgcgc tgcccgccga acccggctcg ctgcccgccg gcgggctcgc gggcagcgcc      660

gccgacctgg tgcggctggg tctgctccac atcgcggagc cgggccgccc ggacctggcc      720

gacccggcgg ccctggggga ggcgacccgg cccacggccg gcgccgaccc gttcgggctg      780

gccgacggct ggggcccggg cctgggccac ttcggccccg cggaggaccg ctggctcggc      840

cacgacggca cgctggacgg cgccacctgt cacctgcgga tccacccccg gcgcggcacc      900

gtcatcgcgc tgaccaccaa ctccccgacc gggcaggccc tgtgggacgc cgtggtcggc      960

gcactgcggg acgcgggcat cgacgtcggc gtgtaccgcc ccgcaccccc gccgtccctc     1020

gcgcagggag gcgcccccgc cggcgcggcg gccttcgcgg actgcacggg cacctaccgc     1080

aacggcgacc tggccgtgac ggtcggcctc gacggcccgc acctcgtact ggaactcccg     1140

ggcggggcaa gggaactggc cgagccgctg gcccggcgga ccttcgccgc ccggggggcc     1200

ggggccttcc tcgggcgctt cgccgtcgac gcgcgcaccg gcgcggtcca cgccctccag     1260

tacagcggcc gcaccctcct gcgggacacg ggccgcgccc acggcgcacg gccgcactaa     1320


<210>  11
<211>  32
<212>  DNA
<213>  Artificial Sequence

<220>
<223>  Forward primer

<400>  11
ccggaattcg tgcacgggga ctcagcggat cc                                  32


<210>  12
<211>  31
<212>  DNA
<213>  Artificial Sequence

<220>
<223>  Reverse primer

<400>  12
cccaagcttt tagtgcggcc gtgcgccgtg g                                   31

## Description

### Technical Field

The present invention relates to a peptide cyclization enzyme capable of producing a macrocyclic peptide, a method for producing the enzyme, and a method for producing a cyclic peptide using the enzyme.

### Background Art

Macrocyclic compounds can impart fixation of the steric conformation of a compound, resistance to a biodegrading enzyme, and the like, and are structural features which are commonly found particularly in physiologically active substances and medicaments derived from natural products. However, there are very limited conventional organic synthetic methods for cyclizing a peptide by efficiently connecting functional groups which are present at a ratio of 1:1 in a molecule and which are located away from each other (see Non Patent Literature 1). Finding an efficient cyclization method is one of problems which have not been solved yet in synthetic organic chemistry.

### Citation List

### Non Patent Literature

Non Patent Literature 1: C. J. White, A. K. Yudin, Nature Chem. 2011, 3, 509-524

### Summary of Invention

### Problem to be Solved by the Invention

An problem to be solved by the present invention is to find a method for cyclizing a peptide by efficiently connecting functional groups which are present at a ratio of 1:1 in a molecule and which are located away from each other.

### Means to Solve the Problem

The present inventors have extensively conducted studies for solving the above-described problem, and found that peptide cyclization enzymes derived from Actinomycetes are capable of efficiently producing a desired macrocyclic peptide, which led to completion of the present invention.

That is, the present invention provides the following (1) to (7).
(1) A peptide cyclization enzyme comprising:
   (a) an amino acid sequence set forth as SEQ ID NO: 1,
   (b) an amino acid sequence having a 35% or more identity to an amino acid sequence set forth as SEQ ID NO: 1, or
   (c) an amino acid sequence set forth as SEQ ID NO: 1 in which one to tens of amino acid residues are deleted, substituted, inserted or added.
(2) A peptide cyclization enzyme comprising an amino acid sequence which is encoded by a DNA containing:
   (a) a nucleotide sequence set forth as SEQ ID NO: 2, or
   (b) a nucleotide sequence which hybridizes under stringent conditions with a nucleotide sequence complementary to a nucleotide sequence set forth as SEQ ID NO: 2.
(3) A DNA comprising:
   (a) a nucleotide sequence encoding an amino acid sequence set forth as SEQ ID NO: 1,
   (b) a nucleotide sequence encoding an amino acid sequence having a 35% or more identity to an amino acid sequence set forth as SEQ ID NO: 1,
   (c) a nucleotide sequence encoding an amino acid sequence set forth as SEQ ID NO: 1 in which one to tens of amino acid residues are deleted, substituted, inserted or added, or
   (d) a nucleotide sequence which hybridizes under stringent conditions with a nucleotide sequence complementary to a nucleotide sequence encoding an amino acid sequence set forth as SEQ ID NO: 1.
(4) A DNA comprising:
   (a) a nucleotide sequence set forth as SEQ ID NO: 2, or
   (b) a nucleotide sequence which hybridizes under stringent conditions with a nucleotide sequence complementary to a nucleotide sequence set forth as SEQ ID NO: 2.
(5) A vector comprising the DNA described in (3) or (4) .
(6) A method for producing a peptide cyclization enzyme, comprising culturing cells into which the DNA described in (3) or (4), or the vector described in (5) has been introduced, and then obtaining a peptide cyclization enzyme from the culture.
(7) A method for producing a cyclic peptide, comprising applying the peptide cyclization enzyme described in (1) or (2) to a substrate peptide.

### Effects of Invention

According to the present invention, there are provided an enzyme capable of efficiently producing a cyclic peptide, particularly a macrocyclic peptide, a method for producing the enzyme, and a method for producing a cyclic peptide using the enzyme. The peptide cyclization enzyme of the present invention has broad substrate specificity, so that it is possible to obtain cyclized peptides having various components and lengths which have been difficult to obtain by conventional organic synthetic methods. According to the present invention, various physiologically active substances and medicaments can be produced.

### Brief Description of Drawings

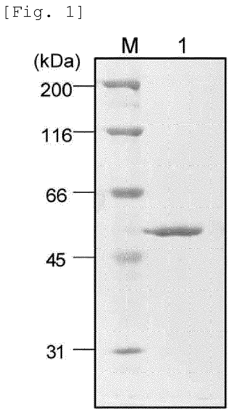

[Figure 1] Figure 1 shows the results of polyacrylamide gel electrophoresis of a peptide cyclization enzyme of the present invention (derived from Streptomyces albidoflavus NBRC 12854). Lane M represents a molecular weight marker, and lane 1 represents the peptide cyclization enzyme of the present invention.

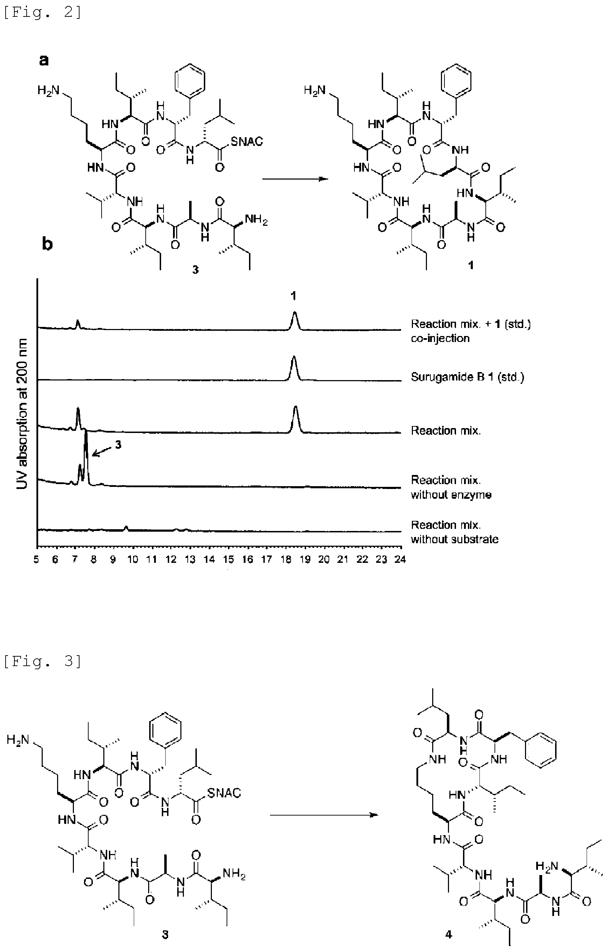

[Figure 2] Figure 2a shows a reaction formula showing conversion of a substrate peptide (3) into a cyclized peptide (1) by the peptide cyclization enzyme of the present invention (derived from Streptomyces albidoflavus NBRC 12854). Figure 2b shows a HPLC chromatogram of a reaction solution when the peptide cyclization enzyme of the present invention is applied to the substrate peptide of Figure 2a. Reaction mix. +1 (std.) co-injection represents a chromatogram of a sample obtained by mixing the reaction solution with a surugamide B preparation indicated with 1 in Figure 2a. Surugamide B1 (std.) represents a chromatogram of the surugamide B preparation. Reaction mix. represents a chromatogram of the reactionsolution. Reaction mix. without enzyme represents a chromatogram of a reaction solution without addition of the peptide cyclization enzyme of the present invention. Reaction mix. without substrate represents a chromatogram of a reaction solution without addition of the substrate peptide.
[Figure 3] Figure 3 shows a reaction formula showing a non-enzymatic cyclization reaction of the substrate peptide.

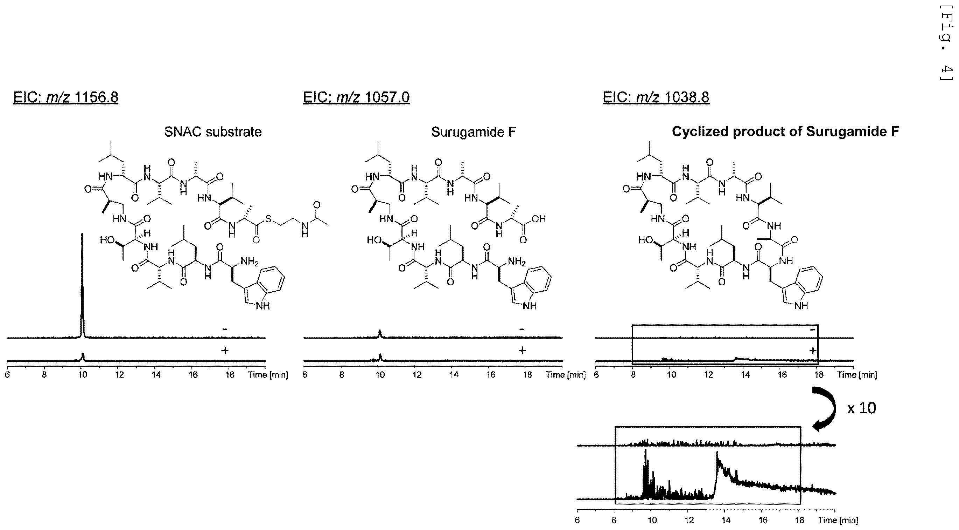

[Figure 4] Figure 4 shows the results of HPLC analysis of a reaction induced by the peptide cyclization enzyme of the present invention (derived from Streptomyces albidoflavus NBRC 12854) when another substrate peptide is used. In the HPLC chromatogram, + represents a chromatogram of a reaction solution with addition of the peptide cyclization enzyme of the present invention, and - represents a chromatogram of a reaction solution without addition of the peptide cyclization enzyme of the present invention. ×10 means that the boxed portion of the chromatogram is magnified by 10 times.

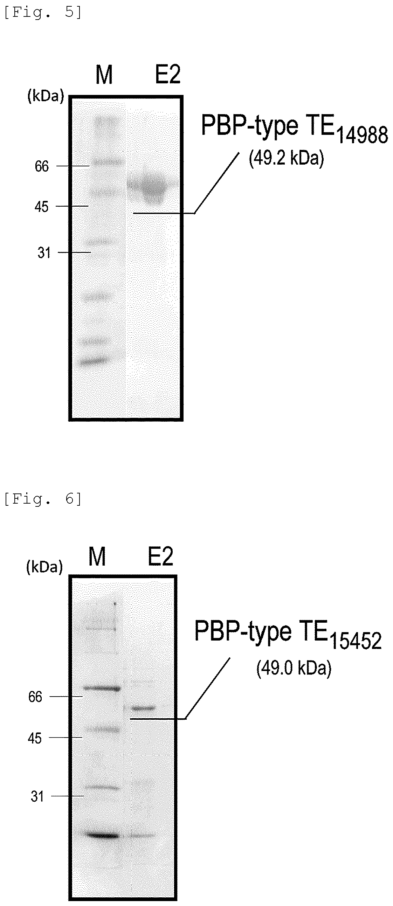

[Figure 5] Figure 5 shows the results of polyacrylamide gel electrophoresis of a peptide cyclization enzyme of the present invention (derived from Goodfellowiella coeruleoviolacea NBRC 14988). Lane M represents a molecular weight marker, and lane E2 represents the peptide cyclization enzyme of the present invention.
[Figure 6] Figure 6 shows the results of polyacrylamide gel electrophoresis of a peptide cyclization enzyme of the present invention (derived from Streptomyces noursei NBRC 15452). Lane M represents a molecular weight marker, and lane E2 represents the peptide cyclization enzyme of the present invention.

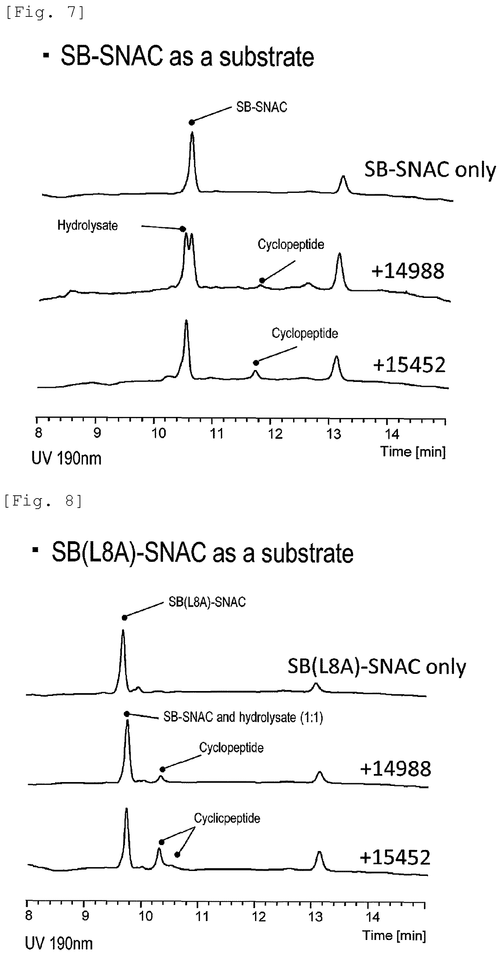

[Figure 7] Figure 7 shows the results of HPLC analysis of cyclization products obtained using the peptide cyclization enzyme derived from Goodfellowiella coeruleoviolacea NBRC 14988 and the peptide cyclization enzyme derived from Streptomyces noursei NBRC 15452 when the substrate peptide 3 (SB-SNAC) shown in Figure 2 is used as a substrate. SB-SNAC only represents a system without addition of an enzyme, +14988 represents a system with addition of the peptide cyclization enzyme derived from Goodfellowiella coeruleoviolacea NBRC 14988, and +15452 represents a system with addition of the peptide cyclization enzyme derived from Streptomyces noursei NBRC 15452.
[Figure 8] Figure 8 shows the results of HPLC analysis of cyclization products obtained using the peptide cyclization enzyme derived from Goodfellowiella coeruleoviolacea NBRC 14988 and the peptide cyclization enzyme derived from Streptomyces noursei NBRC 15452 when as a substrate, a peptide is used in which D-Leu at the C-terminus of the substrate peptide 3 shown in Figure 2 is substituted with D-Ala (SB(L8A)-SNAC). SB(L8A)-SNAC only represents a system without addition of an enzyme, +14988 represents a system with addition of the peptide cyclization enzyme derived from Goodfellowiella coeruleoviolacea NBRC 14988, and +15452 represents a system with addition of the peptide cyclization enzyme derived from Streptomyces noursei NBRC 15452.

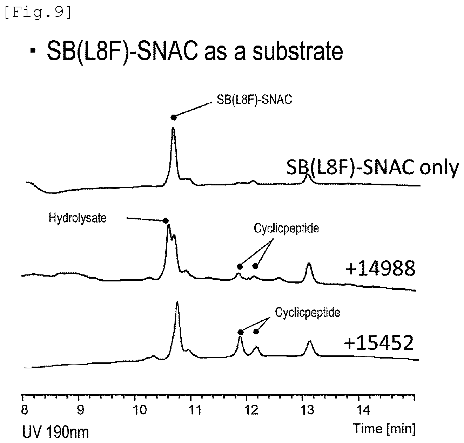

[Figure 9] Figure 9 shows the results of HPLC analysis of cyclization products obtained using the peptide cyclization enzyme derived from Goodfellowiella coeruleoviolacea NBRC 14988 and the peptide cyclization enzyme derived from Streptomyces noursei NBRC 15452 when as a substrate, a peptide is used in which D-Leu at the C-terminus of the substrate peptide 3 shown in Figure 2 is substituted with D-Phe (SB(L8F)-SNAC). SB(L8F)-SNAC only represents a system without addition of an enzyme, +14988 represents a system with addition of the peptide cyclization enzyme derived from Goodfellowiella coeruleoviolacea NBRC 14988, and +15452 represents a system with addition of the peptide cyclization enzyme derived from Streptomyces noursei NBRC 15452.

### Description of Embodiments

In an aspect, the present invention provides a peptide cyclization enzyme comprising:
(a) an amino acid sequence set forth as SEQ ID NO: 1,
(b) an amino acid sequence having a 35% or more identity to an amino acid sequence set forth as SEQ ID NO: 1, or
(c) an amino acid sequence formed by deletion, substitution, insertion or addition of one to tens of amino acid residues in an amino acid sequence set forth as SEQ ID NO: 1.

In the present description, the peptide cyclization enzyme is an enzyme having the action of cyclizing a peptide by binding an amino group, which is present at a portion forming the peptide (e.g. one amino acid residue), to a carboxyl group, which is present at another portion (e.g. another amino acid residue) to generate a peptide bond. The peptide cyclization enzyme of the present invention can cyclize a peptide in a head-to-tail manner, so that a large cyclic peptide can be produced.

In the present description, the amino acid is normally a natural amino acid and an L-isomer, and can encompass nonnatural amino acids such as β-alanine, D-isomer amino acids, and modified amino acids obtained by modifying amino acids by methods such as alkylation, esterification and halogenation.

Unless otherwise specified, the terms as used in the present description have meanings which are normally understood in the fields of biology, biochemistry, chemistry, pharmacy, medicine and the like.

The peptide cyclization enzyme of the present invention may contain an amino acid sequence set forth as SEQ ID NO: 1.

The peptide cyclization enzyme of the present invention may contain a variant sequence of an amino acid sequence set forth as SEQ ID NO: 1. For example, the peptide cyclization enzyme of the present invention may contain an amino acid sequence having an identity of 35% or more, for example 40% or more, preferably 50% or more, for example 60% or more, more preferably 70% or more, for example 80% or more or 85% or more, still more preferably 90% or more, for example 92% or more, 94% or more, 96% or more or 98% or more to an amino acid sequence set forth as SEQ ID NO: 1. The identity of amino acid sequences can be examined by known means such as FASTA search, BLAST search or the like.

When the peptide cyclization enzyme of the present invention contains a variant sequence of an amino acid sequence set forth as SEQ ID NO: 1, it is preferable that in the variant sequence, the amino acid sequence of a portion corresponding to the 63rd to 66th amino acid residues of SEQ ID NO: 1 is Ser-X₁-X₂-Lys, and/or the amino acid sequence of a portion corresponding to the 153rd to 158th amino acid residues of SEQ ID NO: 1 is Ser-Tyr-Ser-Asn-X₃-Gly, and/or the amino acid sequence of a portion corresponding to the 304th to 307th amino acid residues of SEQ ID NO: 1 is Gly-His-X₄-Gly, and/or the amino acid sequence of a portion corresponding to the 374th to 379th amino acid residues of SEQ ID NO: 1 is Gly-X₅-X₆-X₇-Asn-Gly. More preferably, the amino acid sequence of a portion corresponding to the 374th to 379th amino acid residues of SEQ ID NO: 1 is Gly-X₅-X₆-X₇-Asn-Gly. The corresponding portion is not needed to be a portion having same amino acid numbers as the portion of SEQ ID NO: 1, and may a portion near said portion. The corresponding portion can be found by comparing the amino acid sequence with SEQ ID NO: 1. For example, a portion in the amino acid sequence of SEQ ID NO: 5, which corresponds to the 63rd to 66th amino acid residues of SEQ ID NO: 1, is Ser-Leu-Thr-Lys which corresponds to 59th to 62nd amino acid residues. X₁ to X₇ each independently represent an amino acid residue.

For example, the peptide cyclization enzyme of the present invention may contain an amino acid sequence set forth as SEQ ID NO: 1 in which one to several amino acid residues are deleted, substituted, inserted or added. However, the number of amino acid residues submitted to deletion, substitution, insertion or addition in an amino acid sequence set forth as SEQ ID NO: 1 is not limited to one to several, and may be one to tens, preferably 1 to 40, more preferably 1 to 20, still more preferably one to several. The term "tens" means that the number of such amino acid residues may be, for example, 20, 30, 40, 50, 60, 70, 80 or 90. The term "several" means that the number of such amino acid residues may be, for example, 2, 3, 4, 5, 6, 7, 8 or 9. Deletion, substitution, insertion or addition of amino acid residues in an amino acid sequence of protein is known to those skilled in the art. Deletion, substitution, insertion or addition of amino acid residues in an amino acid sequence of the peptide cyclization enzyme of the present invention may be caused by using, for example, a site-specific mutation method or a known chemical method. In substitution of amino acid residues, substitution with homologous amino acids is preferable. The homologous amino acids are known to those skilled in the art.

The peptide cyclization enzyme of the present invention which contains a variant sequence of an amino acid sequence set forth as SEQ ID NO: 1 has a cyclization activity of preferably 50% or more, more preferably 60% or more, still more preferably 70% or more, even more preferably 80% or more, most preferably 90% or more of that of the peptide cyclization enzyme of the present invention which contains an amino acid sequence set forth as SEQ ID NO: 1.

The cyclization activity can be measured by a known method. For example, the amount of a cyclic peptide produced per unit amount of enzyme and unit time may be used as an index of the cyclization activity.

In another aspect, the present invention provides a peptide cyclization enzyme comprising an amino acid sequence which is encoded by a DNA containing:
(a) a nucleotide sequence set forth as SEQ ID NO: 2, or
(b) a nucleotide sequence which hybridizes under stringent conditions with a nucleotide sequence complementary to a nucleotide sequence set forth as SEQ ID NO: 2.

The nucleotide sequence set forth as SEQ ID NO: 2 encodes an amino acid sequence set forth as SEQ ID NO: 1.

In the present description, the nucleotide sequence encompasses degenerate sequences encoding a target amino acid sequence.

The peptide cyclization enzyme of the present invention may contain an amino acid sequence which is encoded by a DNA containing a nucleotide sequence set forth as SEQ ID NO: 2.

The peptide cyclization enzyme of the present invention may contain an amino acid sequence which is encoded by a DNA containing a variant sequence of a nucleotide sequence set forth as SEQ ID NO: 2. For example, the peptide cyclization enzyme of the present invention may contain an amino acid sequence which is encoded by a DNA containing a nucleotide sequence which hybridizes under stringent conditions with a nucleotide sequence complementary to a nucleotide sequence set forth as SEQ ID NO: 2.

The stringent conditions are known to those skilled in the art, and examples thereof include the following conditions:
hybridization is carried out for 16 to 24 hours under the condition of a temperature of 60 to 68°C, preferably 65°C, still more preferably 68°C in a buffer solution containing 0.25 M Na₂HPO₄ (pH 7.2), 7% SDS, 1mM EDTA and a 1 × Denhardt's solution, and washing is performed for 15 minutes twice under the condition of a temperature of 60 to 68°C, preferably 65°C, still more preferably 68°C in a buffer solution containing 20 mM Na₂HPO₄ (pH 7.2), 1% SDS and 1mM EDTA; or
prehybridization is carried out overnight at 42°C in a hybridization solution containing 25% formamide, or 50% formamide as a more severe condition, 4 × SSC (sodium chloride/sodium citrate), 50 mM HEPES (pH 7.0), a 10 × Denhardt's solution and 20 µg/ml modified salmon sperm DNA, and washing is then performed at 37°C in a buffer solution containing 1 × SSC and 0.1% SDS, at 42°C in a buffer solution containing 0.5 × SSC and 0.1% SDS as a more severe condition, or at 65°C in a buffer solution containing 0.2 × SSC and 0.1% SDS as a still more severe condition.

Of course, the stringent conditions are not limited to the above examples.

The peptide cyclization enzyme of the present invention which contains an amino acid sequence which is encoded by a DNA containing a variant sequence of a nucleotide sequence set forth as SEQ ID NO: 2 has a cyclization activity of preferably 50% or more, more preferably 60% or more, still more preferably 70% or more, even more preferably 80% or more, most preferably 90% or more of that of the peptide cyclization enzyme of the present invention which contains an amino acid sequence which is encoded by a DNA containing a nucleotide sequence set forth as SEQ ID NO: 2.

In still another aspect, the present invention provides a DNA comprising:
(a) a nucleotide sequence encoding an amino acid sequence set forth as SEQ ID NO: 1,
(b) a nucleotide sequence encoding an amino acid sequence having a 35% or more identity to an amino acid sequence set forth as SEQ ID NO: 1,
(c) a nucleotide sequence encoding an amino acid sequence set forth as SEQ ID NO: 1 in which one to tens of amino acid residues are deleted, substituted, inserted or added, or
(d) a nucleotide sequence which hybridizes under stringent conditions with a nucleotide sequence complementary to a nucleotide sequence encoding an amino acid sequence set forth as SEQ ID NO: 1.

The identity of amino acid sequences is as described above. The number of amino acid residues submitted to deletion, substitution, insertion or addition is also as described above. The stringent conditions are also as described above.

In still another aspect, the present invention provides a DNA comprising:
(a) a nucleotide sequence set forth as SEQ ID NO: 2, or
(b) a nucleotide sequence which hybridizes under stringent conditions with a nucleotide sequence complementary to a nucleotide sequence set forth as SEQ ID NO: 2.

The stringent conditions are as described above.

The DNA can be obtained by a method known to those skilled in the art. The DNA may be obtained by cloning a surE gene or a homolog or an ortholog thereof. The living organism to be used as a source of the DNA is not particularly limited, and is preferably a microorganism, more preferably Actinomycetes. For example, the DNA of the present invention may be obtained by the method described in Examples of the present description. Examples of the Actinomycetes include, but are not limited to, Streptomyces, Actinomyces, Mycobacterium, Corynebacterium and Goodfellowiella.

The DNA encodes the above-described peptide cyclization enzyme of the present invention. using the DNA, the peptide cyclization enzyme of the present invention can be produced by a genetic engineering method. For example, the peptide cyclization enzyme of the present invention may be obtained from a culture obtained by incorporating the DNA of the present invention into an expression vector, introducing the vector into cells, and culturing the cells. For example, the peptide cyclization enzyme of the present invention may be obtained from a culture obtained by introducing the DNA into cells by use of a known method such as a PEG method, electroporation or a particle gun method, and culturing the cells.

In still another aspect, the present invention provides a vector containing the DNA in the aspect described above. The vector is preferably an expression vector. Various expression vectors are known, and can be appropriately selected and used. Methods for incorporating the DNA of the present invention into a vector are also known.

In still another aspect, the present invention provides a method for producing a peptide cyclization enzyme, comprising culturing cells into which the DNA of the present invention or the vector has been introduced, and then obtaining a peptide cyclization enzyme from the culture. The cells to be used for the method in this aspect are not particularly limited, and may be cells of microorganisms such as bacteria, yeasts, filamentous bacteria or Actinomycetes, plant cells, or animal cells. Examples of preferred cells to be used for this method include, but are not limited to, cells of microorganisms such as Escherichia coli and Bacillus subtilis.

When cells are used which produce the peptide cyclization enzyme of the present invention inside cells, the cells are broken by known means such as ultrasonic waves, a mill or a homogenizer to obtain a liquid extract, from which the peptide cyclization enzyme of the present invention can be obtained by known means such as ammonia sulfate precipitation or chromatography. When cells are used which produce the peptide cyclization enzyme of the present invention outside cells, the peptide cyclization enzyme of the present invention can be obtained by subjecting a culture solution to known means such as ammonia sulfate precipitation or chromatography.

In still another aspect, the present invention provides method for producing a cyclic peptide, comprising applying the peptide cyclization enzyme of the present invention to a substrate peptide.

The peptide cyclization enzyme of the present invention has broad substrate specificity, and can cyclize even a large peptide. Therefore, the composition and the length of the substrate peptide to be used for the method of the present invention are not particularly limited. The substrate peptide may be a target cyclic peptide in which any peptide bond has been cleaved. The substrate peptide may have a length of several or more amino acids, for example 7 or more amino acids, 9 or more amino acids or 11 or more amino acids.

In the case where a cyclic peptide is obtained by using the peptide cyclization enzyme of the present invention, a cyclization reaction is accelerated when the C-terminus and/or the N-terminus of the substrate peptide are a bulky amino acid residue and/or a hydrophobic amino acid residue. Therefore, use of the peptide cyclization enzyme of the present invention enables fusion between bulky amino acid residues, which is difficult by chemical synthesis.

It is preferable from the viewpoint of improving cyclization efficiency that the carboxyl group at C-terminal of the substrate peptide used in the method of the present invention is derivatized and activated. Examples of the derivatization include, but are not limited to, esterification. Examples of the esterification include, but are not limited to, thioesterification, and alkyl-esterification (e.g. methyl-esterification and ethyl-esterification).

By incubating a solution containing the substrate peptide and the peptide cyclization enzyme of the present invention under appropriate conditions, the peptide cyclization enzyme of the present invention can be applied to the substrate peptide. Those skilled in the art can determine the appropriate conditions according to factors such as the type and the concentration of the substrate peptide, and the amount of the enzyme used. Examples of the appropriate conditions include, but are not limited to, those of reaction carried out at room temperature to about 37°C and a pH of 6 to 9 for several hours. The peptide cyclization enzyme may be immobilized to a carrier in use.

Production of the cyclic peptide can be confirmed by known means. For example, a reaction solution may be analyzed using high performance liquid chromatography (HPLC). A product having a molecular weight of the substrate peptide cyclized may be examined using mass spectrometry. The cyclic peptide preparation may be a commercialized product, or may be obtained by chemical synthesis.

The present invention will be described below in a more detailed and specific manner by way of Examples, which should not be construed as limiting the scope of the present invention.

### Example 1

Hereinafter, the peptide cyclization enzyme of the present invention is referred to as "SurE" or "SurE protein".

### (1) Preparation of Plasmid for Expression of Recombinant SurE Protein

With a genome of Streptomyces albidoflavus NBRC 12854 as a template, a gene fragment encoding SurE was amplified by a PCR reaction using a primer SurE_Fw/SurE_Rv as shown below. This was treated with a restriction enzyme EcoRI/HindIII, and inserted into a multi-cloning site (MCS) of pUC19. The inserted sequence was checked, and the inserted fragment was then reinserted into an EcoRI/HindIII site of MCS of pET28 to prepare pET28-surE.
SurE_Fw: CCGGAATTCCATATGGGTGCCGAGGGGGCG (SEQ ID NO: 3)
SurE_Rv: CCCAAGCTTTCAGAGCCGGTGCATGGC (SEQ ID NO: 4)

### (2) Preparation of Recombinant SurE Protein

pET28-surE was introduced into Escherichia coli (E. coli) BL21 to prepare E. coli for expression of recombinant SurE. A single colony of the E. col was inoculated in 2xYT medium containing kanamycin (Km) at 25 µg/ml, and cultured overnight at 37°C. This was inoculated at 1.0% (v/v) in 2xYT medium containing kanamycin (Km) at 25 µg/ml, and cultured at 37°C until OD600 was about 0.5. This was cooled on ice for 5 minutes, ITPG was then added to a final concentration of 0.1 mM, and culturing was performed overnight at 16°C. The culture solution was centrifuged at 4,000 xg, and the bacterial cells were recovered. The bacterial cells were resuspended in a buffer A (20 mM Tris-HCl (pH 8.0), 150 mM, NaCl), and centrifuged at 4,000 xg again. The bacterial cells were resuspended in the buffer A again, and ultrasonically broken. The liquid after cell breakage was centrifuged at 15,000 xg, and the resulting supernatant was subjected to Ni affinity column chromatography equilibrated with a buffer B (20 mM Tris-HCl, 150 mM NaCl, 20 mM imidazole (pH 8.0)) in advance. The resin was further washed with the buffer B, and recombinant SurE protein bound to the resin was then eluted with a buffer C (20 mM Tris-HCl (pH 8.0), 150 mM NaCl, 500 mM imidazole (pH 8.0)). The eluted protein was subjected to polyacrylamide gel electrophoresis. A single band was observed at a molecular weight of 47 kDa as shown in Figure 1. The amino acid sequence of the resulting SurE protein is set forth as SEQ ID NO: 1, and the nucleotide sequence encoding the amino acid sequence is set forth as SEQ ID NO: 2.

### (3) Peptide Cyclization Reaction by Recombinant SurE (i)

A reaction solution having the composition of 20 mM Tris-HCl (pH 8.0), 1 mM substrate peptide and 9 µg of recombinant SurE was prepared using the recombinant SurE protein prepared by the above-described procedure and a substrate peptide (SNAC body indicated with 3 in Figure 2a). The substrate peptide was prepared by fusing N-acetylcysteamine with a peptide synthesized by solid-phase synthesis. The reaction solution was incubated at 30°C for 2 hours, and subjected to HPLC analysis. Here, as a column, COSMOSIL 5C18-MS-II 4.6 × 250 mm Column (nacalai tesque) was used, and as a mobile phase, 41% MeCN + 0.05% TFA was used at a flow rate of 0.8 ml/min. The product was monitored by absorption of UV having a wavelength of 200 nm.

Figure 2b shows the results of the HPLC analysis. The reaction solution of the recombinant SurE protein and the substrate peptide (Reaction mix. in Figure 2b) showed a peak from a single product at a retention time of about 18.4 minutes. The retention time for this peak was identical to the retention time for the peak from a Surugamide B preparation (1 in Figure 2) (Surugamide B1 (Std.) in Figure 2b). A sample obtained by mixing the reaction solution with the Surugamide B preparation showed a single peak at a retention time identical to that for the peak from the Surugamide B preparation (Reaction mix. + 1 (std.) co-injection in Figure 2b). These results revealed that the peptide cyclization enzyme of the present invention (SurE) cyclized the substrate peptide to give a cyclized peptide 1 (Surugamide B) as a single product.

Without use of SurE, the substrate peptide was incubated for 2 days in the presence of Et3N (pH 12), and subjected to HPLC analysis. A peak was observed at a retention time of about 16.2 minutes, which was identical to the retention time for the compound 4 preparation of Figure 3 (data is not shown).

These results showed that the cyclized peptide 1 (Surugamide B) was obtained by the peptide cyclization enzyme of the present invention.

### (4) Peptide Cyclization Reaction by Recombinant SurE (ii)

SurE was applied to the substrate peptide (SNAC body) shown in Figure 4, and the reaction solution was analyzed by HPLC and mass spectrometry (extracted ion chromatogram). Surugamide F was not detected, and a product having a molecular weight corresponding to the molecular weight (m/z 1038.8) of a cyclized product of Surugamide F (cyclic Surugamide F) was detected at a HPLC retention time of about 13.8 minutes. Even when a shorter peptide (7 amino acid residues, SNAC body) and a longer peptide (11 amino acid residues, SNAC body) as compared to the peptide used above were used, a cyclized product was observed to be obtained by the recombinant SurE of the present invention (data not shown). These results showed that the enzyme of the present invention permits a wide range of length of the substrate.

### Example 2

### (1) Cyclization Reaction Using Substrate Having Bulky Amino Acid Residue at C-Terminus/N-Terminus

Experiments were conducted using a substrate in which D-Leu at the C-terminus of the compound indicated in 3 of Figure 2a is substituted with bulkier D-Phe and a substrate in which IIe at the N-terminus of the compound indicated in 3 of Figure 2a is substituted with bulkier L-Trp. Recombinant SurE obtained in Example 1 was used as an enzyme. A reaction was carried out while the concentration of the substrate was changed, the cyclized product was analyzed by HPLC, and a K_{M} value and a kcat value were calculated. The catalytic efficiency was evaluated by using a reaction rate constant kcat/K_{M}.

The kcat/K_{M} value obtained when the above-described two types of substrates were used was two to five times higher than the kcat/K_{M} value obtained when the compound indicated with 3 in Figure 2a was used as a substrate. This result showed that the cyclization reaction was promoted when the amino acid residues at the C-terminus and/or the N-terminus of the substrate were a bulky amino acid residue and/or an amino acid residue having high hydrophobicity. It was shown that use of SurE of the present invention enables a fusion reaction between bulky amino acid residues, which is difficult in chemical synthesis.

### (2) Cyclization Reaction of Recombinant SurE with Methyl Ester as Substrate

A methyl ester which is the compound indicated with 3 in Figure 2a was obtained by extension of a peptide chain by Fmoc solid-phase synthesis, methyl-esterification using Mel, and deprotection of a Boc group. It was confirmed that even when the methyl ester was used as a substrate, the same cyclized product as in the case of using the SNAC body was produced. Since the SurE of the present invention is capable of producing a cyclized product even when a methyl ester which can be supplied more conveniently and inexpensively than the SNAC body is used as a substrate, the SurE of the present invention is considered as an economically advantageous enzyme.

The experimental results shown in Examples above showed that the peptide cyclization enzyme of the present invention was a useful enzyme capable of cyclizing substrate peptides having different compositions and lengths.

### Example 3

### (1) Preparation of Recombinant Peptide Cyclization Enzyme from Microorganisms Other Than Streptomyces albidoflavus

From Goodfellowiella coeruleoviolacea NBRC 14988 and Streptomyces noursei NBRC 15452, cyclization enzymes were obtained in the same manner as in Example 1.

Primers used for cloning were as follows:
forward primer: ccggaattcgtgcccaacgagcaggatctcggg (SEQ ID NO: 7) and reverse primer:
   cccaagctttcatccggtcacctgccgccgc (SEQ ID NO: 8) for Goodfellowiella coeruleoviolacea NBRC 14988; and
forward primer: ccggaattcgtgcacggggactcagcggatcc (SEQ ID NO: 11) and reverse primer:
   cccaagcttttagtgcggccgtgcgccgtgg (SEQ ID NO: 12) for Streptomyces noursei NBRC 15452.

Figures 5 and 6 show the results of polyacrylamide gel electrophoresis of the peptide cyclization enzymes obtained from the above-described two strains of microorganisms. The peptide cyclization enzyme from Goodfellowiella coeruleoviolacea NBRC 14988 showed a single band at 49.2 kDa, while the peptide cyclization enzyme from Streptomyces noursei NBRC 15452 showed a single band at 49.0 kDa.

The amino acid sequence of the peptide cyclization enzyme from Goodfellowiella coeruleoviolacea NBRC 14988 is set forth as SEQ ID NO: 5, and the nucleotide sequence of the peptide cyclization enzyme is set forth as SEQ ID NO: 6. The amino acid sequence of the peptide cyclization enzyme from Streptomyces noursei NBRC 15452 is set forth as SEQ ID NO: 9, and the nucleotide sequence of the peptide cyclization enzyme is set forth as SEQ ID NO: 10. The amino acid sequence of the peptide cyclization enzyme from Goodfellowiella coeruleoviolacea NBRC 14988 had a 38% identity to the amino acid sequence of SEQ ID NO: 1, and the amino acid sequence of the peptide cyclization enzyme from Streptomyces noursei NBRC 15452 had a 37% identity to the amino acid sequence of SEQ ID NO: 1.

### (2) Peptide Cyclization Reaction by Recombinant Peptide Cyclization Enzyme

The two recombinant peptide cyclization enzymes obtained as described above and substrate peptides (SNAC body: "SB-SNAC" indicated with 3 in Figure 2, peptide "SB(L8A)-SNAC" in which D-Leu at the C-terminus of SB-SNAC is substituted with D-Ala, and peptide "SB(L8F)-SNAC" in which D-Leu at the C-terminus of SB-SNAC is substituted with D-Phe) were reacted in the same manner as in Example 1, and the products were observed by HPLC. Figures 7, 8 and 9 show HPLC chromatograms using SB-SNAC as a substrate, using SB(L8A)-SNAC as a substrate, and using SB(L8F)-SNAC as a substrate, respectively.

As is apparent from Figures 7 to 9, the two enzymes produced cyclized peptides from three substrates. In Figure 7, the peak at a retention time of about 11.7 minutes corresponds to a head-to-tail cyclized peptide. In Figure 8, the peak at a retention time of about 10.3 minutes corresponds to a head-to-tail cyclized peptide. In Figure 9, the peak at a retention time of about 11.8 minutes corresponds to a head-to-tail cyclized peptide. In Figures 8 and 9, peaks of two cyclized peptides were observed, and the peak at a longer retention time corresponded to an isopeptide non-enzymatically cyclized between a lysine residue and an amino acid residue at the C-terminus in the substrate peptide.

From these results, it was confirmed that a peptide cyclization enzyme was obtained even from microorganisms other than Streptomyces albidoflavus.

### Industrial Applicability

The present invention is applicable to production of medicaments and laboratory reagents, etc.

### Sequence Listing Free Text

SEQ ID NO: 1 shows an amino acid sequence of the peptide cyclization enzyme of the present invention (derived from Streptomyces albidoflavus NBRC 12854).
SEQ ID NO: 2 shows a nucleotide sequence encoding the peptide cyclization enzyme of the present invention (derived from Streptomyces albidoflavus NBRC 12854).
SEQ ID NO: 3 shows a nucleotide sequence of the forward primer used for cloning of the peptide cyclization enzyme of the present invention (derived from Streptomyces albidoflavus NBRC 12854).
SEQ ID NO: 4 shows a nucleotide sequence of the reverse primer used for cloning of the peptide cyclization enzyme of the present invention (derived from Streptomyces albidoflavus NBRC 12854).
SEQ ID NO: 5 shows an amino acid sequence of the peptide cyclization enzyme of the present invention (derived from Goodfellowiella coeruleoviolacea NBRC 14988).
SEQ ID NO: 6 shows a nucleotide sequence of the peptide cyclization enzyme of the present invention (derived from Goodfellowiella coeruleoviolacea NBRC 14988).
SEQ ID NO: 7 shows a nucleotide sequence of the forward primer used for cloning of the peptide cyclization enzyme of the present invention (derived from Goodfellowiella coeruleoviolacea NBRC 14988).
SEQ ID NO: 8 shows a nucleotide sequence of the reverse primer used for cloning of the peptide cyclization enzyme of the present invention (derived from Goodfellowiella coeruleoviolacea NBRC 14988).
SEQ ID NO: 9 shows an amino acid sequence of the peptide cyclization enzyme of the present invention (derived from Streptomyces noursei NBRC 15452).
SEQ ID NO: 10 shows a nucleotide sequence encoding the peptide cyclization enzyme of the present invention (derived from Streptomyces noursei NBRC 15452).
SEQ ID NO: 11 shows a nucleotide sequence of the forward primer used for cloning of the peptide cyclization enzyme of the present invention (derived from Streptomyces noursei NBRC 15452).
SEQ ID NO: 12 shows a nucleotide sequence of the reverse primer used for cloning of the peptide cyclization enzyme of the present invention (derived from Streptomyces noursei NBRC 15452).

## Claims

1. A peptide cyclization enzyme comprising:
(a) an amino acid sequence set forth as SEQ ID NO: 1,
(b) an amino acid sequence having a 35% or more identity to an amino acid sequence set forth as SEQ ID NO: 1, or
(c) an amino acid sequence set forth as SEQ ID NO: 1 in which one to tens of amino acid residues are deleted, substituted, inserted or added.

2. A peptide cyclization enzyme comprising an amino acid sequence which is encoded by a DNA containing:
(a) a nucleotide sequence set forth as SEQ ID NO: 2, or
(b) a nucleotide sequence which hybridizes under stringent conditions with a nucleotide sequence complementary to a nucleotide sequence set forth as SEQ ID NO: 2.

3. A DNA comprising:
(a) a nucleotide sequence encoding an amino acid sequence set forth as SEQ ID NO: 1,
(b) a nucleotide sequence encoding an amino acid sequence having a 35% or more identity to an amino acid sequence set forth as SEQ ID NO: 1,
(c) a nucleotide sequence encoding an amino acid sequence set forth as SEQ ID NO: 1 in which one to tens of amino acid residues are deleted, substituted, inserted or added, or
(d) a nucleotide sequence which hybridizes under stringent conditions with a nucleotide sequence complementary to a nucleotide sequence encoding an amino acid sequence set forth as SEQ ID NO: 1.

4. A DNA comprising:
(a) a nucleotide sequence set forth as SEQ ID NO: 2, or
(b) a nucleotide sequence which hybridizes under stringent conditions with a nucleotide sequence complementary to a nucleotide sequence set forth as SEQ ID NO: 2.

5. A vector comprising the DNA according to claim 3 or 4.

6. A method for producing a peptide cyclization enzyme, comprising culturing cells into which the DNA according to claim 3 or 4, or the vector according to claim 5 has been introduced, and then obtaining a peptide cyclization enzyme from the culture.

7. A method for producing a cyclic peptide, comprising applying the peptide cyclization enzyme according to claim 1 or 2 to a substrate peptide.
